# EUROPEAN PATENT APPLICATION

(11) **EP 1 346 720 A2**
(43) Date of publication of application: **24.09.2003**
(21) Application number: 03290692.7
(22) Date of filing: 19.03.2003
(51) Int. Cl.: A61K 7/46, A61K 7/13, A61K 7/135

(54) **Composition for deodorizing hair dyeing and hair bleaching compositions**

(30) Priority: 22.03.2002 JP 2002080167
(71) Applicant: Takasago International Corporation, Tokyo-to 144-8721 (JP)
(72) Inventor: Sakurai, Kazutoshi., c/o Takasago I.C.,Central R.L, Hiratsuka-shi., Kanagawa254-0073 (JP); Miyasaka, Masataka., c/o Takasago I.C.,C.R.L., Kanagawa 254-0073 (JP); Nagano, Junko, Takasago I.C.,C.R.L., Kanagawa 254-0073 (JP)
(74) Representative: Uchida, Kenji

(57) **Abstract**

A deodorant composition which has a superior effect in masking unpleasant smells originated from components such as ammonia formulated in compositions for hair color and compositions for hair bleach and which is also stable in alkaline products and acidic solution products. A deodorant composition is prepared by adding at least one compound or essential oil selected from Koavone, linalool oxide, rosephenone, Sandalore, Sandalmysore core, Bacdanol, Kephalis, cis-p-menthan-7-ol, nerolidol, raspberry ketone, α,3,3-trimethylcyclohexanemethyl formate, p-methoxyphenethyl alcohol, 2,2,6-trimethylcyclohexanecarboxylic acid ethyl ester, 2,2,6-trimethyl-1-crotonylcyclohexane, tansy oil and basil oil.

## Description

### FIELD OF THE INVENTION

This invention relates to a deodorant composition for hair color, which has a superior effect to mask unpleasant smells originated from substances such as ammonia and is stable in alkaline and acidic solutions. It also relates to a deodorant composition for hair bleach, which has a superior effect to mask unpleasant smells originated from substances such as ammonia and is stable in alkaline and acidic solutions. The invention further relates to a hair color composition and a hair bleach composition, which comprise the respective deodorant compositions.

### BACKGROUND OF THE INVENTION

Each of many hair dyes is constituted from two types of preparations called a first preparation (to be referred sometimes to as "hair color first preparation" hereinafter) and a second preparation (to be referred sometimes to as "hair color second preparation" hereinafter). The first preparation contains dye intermediates including, e.g., an alkaline agent such as ammonia or alkanolamine and a surfactant, and the second preparation contains, e.g., an oxidizing agent such as hydrogen peroxide and a pH adjusting agent. The main role of the first preparation is to effect penetration of the dye intermediates and oxidizing agent into hair through swelling of hair by the alkaline agent, and the main role of the second preparation is to remove melanin pigment from hair, develop a color through mutual binding of molecules of the dye intermediates and thereby fix the pigment into hair.

Various deodorant compositions have been examined in order to mask unpleasant smells originated from the smells such as of ammonia and alkanolamine formulated in the first preparation. However, since the first preparation generally shows an alkalinity of from pH 8 to 11 due to large amount of the formulated ammonia, it is required that a fragrance to be formulated has a property of being stable to the alkaline nature.

To date, stability and masking effect of various fragrance components have been examined, and deodorant compositions containing specified fragrances have been reported. For example, it has been reported in JP-A-2000-344629 that 17 kinds of fragrance compounds have deodorization effects. In this case, a fragrance composition prepared by blending one or more of these fragrances is added to an alkali solution of the first preparation, and masking of the ammoniacal smell is evaluated when the filling bottle of the first preparation is opened and during the hair dye treatment.

Though the 17 fragrance compounds rendered possible exertion of a reasonable stabilizing effect, concern has been directed toward a fragrance which is further stable and has more higher masking effect.

On the other hand, the second preparation contains an oxidizing agent such as hydrogen peroxide and is adjusted to an acidic pH value of approximately from 2 to 4, and a fragrance is rarely used therein in general.

However, since the first preparation and second preparation are mixed just before use, not only masking in the products before mixing but also the masking effect just after mixing and during hair treatment thereafter and the masking effect after the hair treatment become important points.

Accordingly, concern has been directed toward the advent of a fragrance which shows stabilizing effect in the acidic second preparation and has high masking effect for the ammoniacal smell when hair are treated by mixing the first preparation and second preparation, as a common means at the time of hair color treatment, and the smells originated from other compounds such as alkanolamines and sulfite which are used as the components that constitute the base material.

On the other hand, known hair bleaches include a single preparation-type hair bleach which contains an oxidizing agent such as hydrogen peroxide as the main component and a double preparation-type hair bleach which, similar to the case of the hair dye, is constituted by a first preparation containing an alkaline agent such as ammonia (to be referred sometimes to as "hair bleach first preparation" hereinafter) and a second preparation containing an oxidizing agent such as hydrogen peroxide (to be referred sometimes to as "hair bleach second preparation" hereinafter).

Since bleaching action of the single preparation type hair bleach is weak, the double preparation type hair bleach which, similar to the case of the hair dye, is used in treating hair by mixing the first preparation and second preparation just before use is frequently used.

Accordingly, similar to the case of the hair dye, concern has been directed toward the advent of a fragrance effective for a hair bleach containing compounds which generate ammoniacal smell and alkanolamine-derived strong smell, namely a fragrance which shows high masking effect for the ammoniacal smell and the smells originated from other compounds such as alkanolamines and sulfite which are used as the components that constitute the base material.

### SUMMARY OF THE INVENTION

The present invention contemplates providing a fragrance which can be used in the hair dye or the hair color first preparation and hair color second preparation. That is, it is to provide a fragrance composition, in which the used fragrance components show excellent effect to mask ammoniacal stimulating smell when formulated in the hair color first preparation and are also stable in the base material and show excellent effect to mask the base material smell when formulated in the hair color second preparation, and which renders possible masking of various smells generated during decoloration of hair and hair dye treatment starting from the generation of oxygen due to decomposition of hydrogen peroxide after mixing with the hair color first preparation and also retaining of desirable odor after the treatment. Also to be provided is a deodorant composition for hair color, which has excellent effect in masking smells during a period of from before the hair color treatment to after the treatment. Further to be provided is a deodorant composition for hair bleach, which is stable in a hair bleach or in the hair bleach first preparation and hair bleach second preparation and has excellent effect in masking smells in these hair bleaches and during a period of from before the hair bleach treatment to after the treatment.

In other words, an object of the present invention is to find a deodorant composition which is stable in an alkaline aqueous solution having a pH value of from 8 to 11 and a hydrogen oxide-containing acidic solution having a pH value of from 2 to 4, without causing decoloration and changes in aroma for a prolonged period of time, and has markedly excellent effect in masking ammoniacal smell.

With the aim of solving above problems, the present inventors have conducted intensive studies and found as a result that a specified fragrance is stable in alkaline and acidic solutions, has excellent masking effect and retains a desirable odor for a while after hair dye treatment, and have reached the present invention by further continuing the studies.

Accordingly, the present invention provides a deodorant composition for hair color, which comprises a composition containing at least one compound or essential oil selected from acetyl diisoamylene, linalool oxide, rosephenone, 2-methyl-4-(2,2,3-trimethyl-3-cyclopenten-1-yl)-2-buten-1-ol, 3-methyl-5-(2,2,3-trimethyl-3-cyclopenten-1-yl)-pentan-2-ol, 2-ethyl-4-(2,2,3-trimethyl-3-cyclopenten-1-yl)-2-buten-1-ol, 4-(1-ethoxyvinyl)-3,3,5,5-tetramethylcyclohexanone, cis-p-menthan-7-ol, nerolidol, raspberry ketone, α,3,3-trimethylcyclohexanemethyl formate, p-methoxyphenethyl alcohol, 2,2,6-trimethylcyclohexanecarboxylic acid ethyl ester, 2,2,6-trimethyl-1-crotonylcyclohexane, tansy oil and basil oil,
also a deodorant for hair bleach, which contains at least one species selected from these compounds or essential oils, and
a hair color composition and a hair bleach composition, which respectively comprises these deodorant compositions.

### DETAILED DESCRIPTION OF THE INVENTION

The following describes the present invention in detail.

Unless otherwise indicated, all parts, percentages, ratios, etc. used in this specification are by weight.

Many of the specified fragrances to be used in the deodorant composition of the present invention are already known and can be easily obtained by purchasing commercial products. A part of the fragrances can also be synthesized.

That is, fragrances such as acetyl diisoamylene (Koavone, a trade name of IFF), linalool oxide, rosephenone, 2-methyl-4-(2,2,3-trimethyl-3-cyclopenten-1-yl)-2-buten-1-ol (Sandalmysore core, a trade name of Kao Corp.), 3-methyl-5-(2,2,3-trimethyl-3-cyclopenten-1-yl)-pentan-2-ol (Sandalore, a trade name of Givaudan), 2-ethyl-4-(2,2,3-trimethyl-3-cyclopenten-1-yl)-2-buten-1-ol (Bacdanol, a trade name of IFF), 4-(1-ethoxyvinyl)-3,3,5,5-tetramethylcyclohexanone (Kephalis, a trade name of Givaudan), cis-p-menthan-7-ol (Mayol, a trade name of Firmenich), nerolidol, raspberry ketone, α,3,3-trimethylcyclohexanemethyl formate (Apharmate, a trade name of IFF), p-methoxyphenethyl alcohol, 2,2,6-trimethylcyclohexanecarboxylic acid ethyl ester (Thesaron, a trade name of Takasago International Corporation) and 2,2,6-trimethyl-1-crotonylcyclohexane (Dihydrodamascone, produced by Takasago International Corporation) can be easily obtained by purchasing commercial products. Also, linalool oxide, rosephenone, nerolidol and raspberry ketone may be prepared personally. In addition, tansy oil and basil oil can also be easily obtained by purchasing commercial products. These fragrances may be racemic compounds or optically active substances in response to uses.

These fragrances show excellent effect in masking unpleasant smells caused by ammonia and its derivatives formulated particularly in the hair color first preparation or hair bleach first preparation and are also excellent in view of aromatic stability and chemical stability. Also, they are stable in liquid under acidic condition and show excellent effect in masking various unpleasant smells.

Particularly in Thesaron and Dihydrodamascone among these fragrances, the presence of stereoisomers (cis and trans) for the cyclohexane ring is known in view of their chemical structures, and the presence of many optically active substances having different optical activities is also known due to the presence of asymmetric carbon atoms. These stereoisomers and optically active substances are slightly different in terms of aroma and stability, but their aroma and stability are generally excellent in comparison with other fragrances. Thus, since their stereoisomers and optically active substances can be optionally selected in response to their use, a racemic body mixture may be used as such or a commercially available optically active substance may be used after all according to the present invention.

Further desirable results can be obtained when two or more of the compounds or essential oils defined by the present invention are used in combination. For example, a composition containing a compound or essential oil as a component A selected from acetyl diisoamylene, linalool oxide, rosephenone, 2-methyl-4-(2,2,3-trimethyl-3-cyclopenten-1-yl)-2-buten-1-ol, 3-methyl-5-(2,2,3-trimethyl-3-cyclopenten-1-yl)-pentan-2-ol, 2-ethyl-4-(2,2,3-trimethyl-3-cyclopenten-1-yl)-2-buten-1-ol, 4-(1-ethoxyvinyl)-3,3,5,5-tetramethylcyclohexanone, cis-p-menthan-7-ol, nerolidol, raspberry ketone, α,3,3-trimethylcyclohexanemethyl formate, p-methoxyphenethyl alcohol, tansy oil and basil oil, and a compound as a component B selected from 2,2,6-trimethylcyclohexanecarboxylic acid ethyl ester (Thesaron, mfd. by Takasago International Corporation) and 2,2,6-trimethyl-1-crotonylcyclohexane (Dihydrodamascone) shows excellent effect in masking unpleasant smells caused by ammonia and its derivatives and is also excellent in view of aromatic stability and chemical stability.

Though blending ratio of these component A and component B is not particularly limited, it is desirable that the blending ratio of the component A and component B is, e.g., from 99.5:0.5 to 10:90 (weight ratio), more desirably from 95.5:5 to 20:80 (weight ratio).

In addition to the fragrance components described above, the deodorant composition of the present invention may further contain appropriate component(s) in compliance with the objects and types of the deodorant composition. Examples of the additional components include fragrance components other than those described above, diluents, solvents, and the like.

Examples of the other fragrance components include terpene hydrocarbons such as p-cymene, terpinolene and myrcene, aldehydes such as heptanal, octanal, benzaldehyde, salicylic aldehyde, citronellal and alpha-hexylcinnamic aldehyde, esters such as methyl jasmonate, gamma-nonalactone, gamma-decalactone and cumarin, ethers such as anisole, paracresyl methyl ether, beta-naphthol methyl ether and beta-naphthol ethyl ether, and ketones such as menthone, acetophenone, alpha-damascone, beta-damascone, α-ionone, β-ionone, methylionone, irone and dihydrojasmone. Also included are alcohols such as isopropyl alcohol, cis-3-hexenol, heptanol, 2-octanol, benzyl alcohol, citronellol, geraniol, terpineol, tetrahydrogeraniol, anise alcohol and phenethyl alcohol.

Also, as the diluent and solvent, it is desirable to use dipropylene glycol or 3-methoxy-3-propanol or the like from the general purpose point of view.

It is possible to improve masking of various types of unpleasant smell originating from each of the base materials by mixing the deodorant composition of the present invention with a hair color composition or a hair bleach composition. That is, it can be used by optionally mixing with not only a hair color first preparation or a hair bleach first preparation which uses large amounts of ammonia and alkanolamines as a matter of course, but also with hair care products such as hair dye pretreatment shampoo, shampoo for after-hair dye treatment, hair rinse, hair treatment, hair cream, hair lotion and hair foam. It can also be mixed with a hair color second preparation, or the second preparation of a double preparation type or triple preparation type hair bleach. In this connection, the hair color composition according to the present invention means the hair color first preparation and hair color second preparation, as well as hair dyes, and the hair bleach composition according to the present invention means the hair bleach first preparation and hair bleach second preparation, as well as hair bleaches.

The deodorant composition of the invention is blended at an amount of preferably 0.01 to 30 % by weight, particularly preferably 0.1 to 1.0 % by weight based on the total amount of the first hair color agent. Further, the same is true with the second hair color agent, the first hair bleach agent and the second hair bleach agent.

The deodorant composition of the invention may be blended at an amount of 0.01 to 30 % by weight, particularly 0.1 to 2.0 % by weight to the total amount of a hair treatment composition. In case of two agent types such as two agent-type hair colors and two agent-type hair bleaches, for example, the deodorant composition of the invention may be blended at an amount of 0.01 to 30% by weight, particularly 0.1 to 2.0% by weight based on the total amount of a first agent and a second agent. In that case, the deodorant composition of the invention may satisfactorily be blended in either one or both of the first agent and the second agent.

It is particularly desirable to use it by formulating in a hair treating preparation which contains ammonia, such as a hair color first preparation or hair bleach first preparation, because it can mask the unpleasant smell originated from ammonia released from the products and the unpleasant smell during the use of hair dyes and after the hair treatment.

A permeation-promoting agent is added to the deodorant composition of the invention, to prepare a hair color composition or a hair bleach composition, specifically a first hair color agent or a first hair bleach agent. In case of hair color composition, a dye is additionally blended in the resulting mixture to prepare a first hair color agent.

Furthermore, an oxidizing agent is added to the deodorant composition of the invention, to prepare a hair color composition or a hair bleach composition, specifically a second hair color agent or a second hair bleach agent.

Such two agent-type hair color composition and such two agent-type hair bleach composition, which contain these first agents and second agents, are also the inventive products.

As the permeation-promoting agent to be blended in the hair color composition or the hair bleach composition of the invention, for example, ammonia, aqueous ammonia, monoethanolamine, triethanolamine, isopropanolamine, potassium hydroxide and sodium hydroxide are used.

The content of the permeation-promoting agent in such first agent is not particularly limited and is preferably from 0.01 to 20% by weight, particularly preferably 0.1 to 15% by weight based on the total amount of such first agent.

Typically, the first agent is adjusted to preferably pH 7 to 12, particularly preferably pH 8 to 11.

The dye to be used in the hair color composition of the invention includes for example oxidative dyes.

The types of the oxidative dyes include for example but are not limited to p-phenylenediamine, p-phenylenediamine hydrochloride, m-phenylenediamine, o-phenylenediamine, toluene-2,5-diamine, resorcin, p-nitro-o-phenylenediamine, p-nitro-m-phenylenediamine sulfate, p-aminophenol, m-aminophenol, o-aminophenol, 2-amino-4-nitrophenol, 2-amino-5-nitrophenol, 5-amino-o-cresol, 2,4-diaminophenoxyethanol hydrochloride, 2,6-diaminopyridine, and 1,5-dihydroxynaphthalene.

The content of the dye in the first agent is not particularly limited and is preferably from 0.01 to 15% by weight based on the total amount of the first agent.

In case of the hair bleach composition in accordance with the invention, a first agent is used without an oxidative dye.

Examples of the oxidizing agent to be contained in the second agent includes hydrogen peroxide and urea peroxide. The content thereof is not particularly limited and is preferably from 0.1 to 15% by weight, particularly preferably from 1 to 10% by weight based on the total amount of the second agent.

Typically, the second agent is adjusted to preferably pH 2 to 4, particularly preferably pH 2 to 3.

The hair color composition and the hair bleach composition in accordance with the invention may appropriately be blended with other additives such as nonionic surfactants; anionic surfactants such as alkyl sulfate ester salt and polyoxyethylene alkyl ether sulfate salt; cationic surfactants; ampholytic surfactants such as alkyldimethylaminoacetate betaine; moisturizers such as glycerin and propylene glycol; oily ingredients; stabilizers; thickeners such as carboxymethyl cellulose, hydroxyethyl cellulose, and hydroxypropyl cellulose; pH adjusters such as phosphoric acid, hydrochloric acid, phosphate dihydrogen monopotassium, citric acid and succinic acid; chelating agents such as ethylenediaminetetraacetic acid; anti-oxidants; and preservatives.

For the hair color composition and the hair bleach composition in accordance with the invention, further, media such as water and/or organic solvents may be used. The organic solvents include ethanol, benzyl alcohol, propylene glycol, diethylene glycol and glycerin.

The hair color composition and the hair bleach composition in accordance with the invention can be formulated into, for example, liquid, cream, gel, paste, mousse, solid, aerosol, foam and powder.

The hair color composition and the color bleach composition of the invention are used, after the first agent and the second agent of each of the compositions are mixed together.

For decoloring or coloring using the hair bleach composition or the hair color composition of the invention, for example, the hair color composition and the hair bleach composition of the invention may be applied to hair and left as they are. Subsequently, the hair color composition and the hair bleach composition may be rinsed off. The resulting hair may be dried.

The deodorant composition of the present invention for hair color shows superior effect in masking the unpleasant smell originated from ammonia when mixed with the hair color first preparation and also shows excellent aromatic stability under a relatively high temperature condition of 45°C for a prolonged period of 3 months, is stable also in the hair color second preparation, and shows superior effect in masking the unpleasant smell originated from ammonia and other unpleasant smells even after treatment of hair with the hair color first preparation and hair color second preparation.

In addition, the deodorant composition of the present invention for hair bleach shows superior effect in masking the unpleasant smell originated from ammonia when mixed with a double preparation type hair bleach first preparation and also shows excellent aromatic stability at 45°C for a prolonged period of 3 months, similar to the case of the deodorant composition for hair color. Also, it is stable in the hair bleach second preparation as well, and shows superior effect in masking the unpleasant smell originated from ammonia and other unpleasant smells even after treatment of hair by mixing with the first preparation and second preparation.

The following describes the present invention further in detail with reference to Examples, but the present invention is not restricted by these Examples.

### Example 1

A hair color first preparation was prepared based on the formulation shown in Table 1. In this connection, illustrative example of the deodorant composition in Table 1 is shown in Table 2.

**Table 1**

| Compounds | Weight % |
|---|---|
| Toluene-2,5-diamine aqueous solution (20%) | 7.0 |
| p-Aminophenol | 2.0 |
| m-Dihydroxybenzene | 0.4 |
| Anhydrous sodium sulfite | 0.4 |
| Disodium edetate | 0.3 |
| Aqueous ammonia (28%) | 7.0 |
| Ammonium chloride | 3.5 |
| Ethanol (95%) | 15.0 |
| Lauric acid | 5.0 |
| Diethanolamide laurate | 14.5 |
| Polyoxyethylene (20) octyldodecyl ether | 10.0 |
| Propylene glycol | 10.0 |
| Deodorant composition | 0.2 |
| Purified water | balance |

(Evaluation) Effect of the hair color first preparation to mask the unpleasant smell originated from ammoniacal smell and stability of its aroma were evaluated by the following evaluation method.

### (Evaluation method)

### (1) Masking effect on the unpleasant smell originated from ammoniacal smell

The hair color first preparation was stored at 37°C for 12 weeks, and then its unpleasant smell-masking effect was evaluated by sensory test based on the following criteria.
A; very good, B; slightly good, C; usual or slightly bad, D; bad

### (2) Stability of aroma and masking effect

A hair dye treatment was carried out by a conventional method using the hair color first preparation.

(2-a) Stability effect: Changing degree of the aromatic tone at the bottle mouth when the container was opened before the treatment was evaluated by sensory test based on the following criteria.
A; no change, B; slightly changed, C; fairly changed, D; greatly changed

(2-b) Masking effect: A smell from hair during the treatment (just after application to hair) and after the treatment was evaluated by sensory test based on the following criteria.
A; very good, B; slightly good, C; usual or slightly bad, D; bad

Results of the evaluation by the evaluation methods are shown in Table 2. In this connection, the "masking effect (1)" in the table is an evaluation result by the evaluation method (1), the "stability of aroma" is an evaluation result by the evaluation method (2-a), and other "masking effect" is an evaluation result by the evaluation method (2-b).

**Table 2**

| Deodorant composition (fragrance 100%) | Masking Effect (1) | Stability of aroma | Masking effect | |
|---|---|---|---|---|
| | | | during treatment | after treatment |
| Acetyl diisoamylene | A | A | A | A |
| Linalool oxide | A | A | A | A |
| Rosephenone | A | A | A | A |
| Sandalmysore core (Kao) | A | A | A | A |
| Sandalore (Givaudan) | A | A | A | A |
| Bacdanol (IFF) | A | A | A | A |
| Levosandol (Takasago) | A | A | A | A |
| Kephalis (Givaudan) | A | A | A | A |
| Mayol (Firmenich) | A | A | A | A |
| Nerolidol | A | A | A | A |
| Raspberry ketone | A | A | A | A |
| Apharmate (IFF) | A | A | A | A |
| p-Methoxyphenethyl alcohol | A | A | A | A |
| Thesaron (Takasago) | A | A | A | A |
| Dihydrodamascone | A | A | A | A |
| Tansy oil | A | A | A | A |
| Basil oil | A | A | A | A |
| levosandol: (E)-(R)-2-ethyl-4-(2,2,3-trimethyl-3-cyclopenten-1-yl)-2-buten-1-ol | | | | |

As is evident from the results of Table 2, it was revealed that all preparations of the present invention are excellent in terms of stability, have high masking effect and exert superior effects even during the hair treatment or after the treatment.

### Example 2

A hair color first preparation containing the deodorant composition shown in Table 3 was obtained based on the formulation shown in Table 1.

**Table 3**

| Deodorant composition (mixed fragrances) | Weight % |
|---|---|
| Levosandol (Takasago) | 2.0 |
| Rosephenone | 5.0 |
| Linalool oxide | 1.0 |
| Kephalis (Givaudan) | 5.0 |
| Mayol (Firmenich) | 5.5 |
| Nerolidol | 8.0 |
| Raspberry ketone | 0.5 |
| Apharmate (IFF) | 2.5 |
| p-Methoxyphenethyl alcohol | 10.0 |
| Tansy oil | 10.0 |
| Methyl ionone | 15.0 |
| Delfon (Firmenich) | 0.5 |
| Tetrahydrolinalool | 15.0 |
| Musk T (Takasago) | 5.0 |
| Ethyl-2-methyl butyrate | 0.5 |
| Thesaron (Takasago) | 2.0 |
| Dihydrodamascone | 0.5 |
| Dipropylene glycol | 7.0 |

(Evaluation) Effect of the hair color first preparation to mask the unpleasant smell originated from ammoniacal smell and stability of its aroma were evaluated in the same manner as in Example 1. That is, (1) masking effect on the unpleasant smell originated from ammoniacal smell, (2-a) stability effect when a hair treatment was carried out by a conventional method using the hair color first preparation and (2-b) masking effect when a hair treatment was carried out by a conventional method using the hair color first preparation were evaluated in the same manner as in Example 1.

The results are shown in Table 4.

**Table 4**

| | (1) | (2-a) | (2-b) |
|---|---|---|---|
| Fragrance mixture | A | A | A |

As is evident from the results shown in Table 4, the deodorant composition containing the compound of the present invention is excellent in stability and has high masking effect. Particularly in (2-b), it was valued that aroma from hair during treatment (just after application to hair) and after treatment was vary good in both cases when a hair treatment was carried out by a conventional method using the hair color first preparation.

### Example 3

A hair bleach first preparation was prepared by the formulation shown in Table 5. In this connection, illustrative examples of the deodorant composition in Table 5 are shown in Table 6.

**Table 5**

| Compound | Weight % |
|---|---|
| Anhydrous sodium sulfite | 0.4 |
| Disodium edetate | 0.3 |
| Aqueous ammonia (28%) | 7.0 |
| Ammonium chloride | 3.5 |
| Ethanol (95%) | 15.0 |
| Lauric acid | 5.0 |
| Diethanolamide laurate | 13.5 |
| Polyoxyethylene (20) octyldodecyl ether | 10.0 |
| Propylene glycol | 10.0 |
| Distearyldimethylammonium chloride | 1.5 |
| Dimethyl polysiloxane | 1.5 |
| Deodorant composition | 0.2 |
| Purified water | balance |

(Evaluation) Effect of the hair bleach first preparation to mask the unpleasant smell originated from ammoniacal smell and stability of its aroma were evaluated by the following evaluation method.

### (Evaluation method)

### (3) Masking effect on the unpleasant smell originated from ammoniacal smell

The hair bleach first preparation was stored at 37°C for 12 weeks, and then its unpleasant smell-masking effect was evaluated by a sensory test based on the following criteria.
A; very good, B; good, C; usual or slightly bad, D; bad

### (4) Stability of aroma and masking effect

A bleaching treatment was carried out by a conventional method using the hair bleach first preparation.

(4-a) Stability effect: Changing degree of the aromatic tone at the bottle mouth when the container was opened before the treatment was evaluated by a sensory test based on the following criteria.
A; no change, B; slightly changed, C; fairly changed, D; greatly changed

(4-b) Masking effect: A smell from hair during the treatment (just after application to hair) and after the treatment was evaluated by a sensory test based on the following criteria.
A; very good, B; slightly good, C; usual or slightly bad, D; bad

Results of the evaluation by the evaluation methods are shown in Table 6. In this connection, the "masking effect (3)" in the table is an evaluation result by the evaluation method (3), the "stability of aroma" is an evaluation result by the evaluation method (4-a), and other "masking effect" is an evaluation result by the evaluation method (4-b).

**Table 6**

| Compound | Masking Effect (3) | Stability of aroma | Masking effect | |
|---|---|---|---|---|
| | | | during treatment | after treatment |
| Koavone (IFF) | A | A | A | A |
| Linalool oxide | A | A | A | A |
| Rosephenone | A | A | A | A |
| Sandalmysore core (Kao) | A | A | A | A |
| Sandalore (Givaudan) | A | A | A | A |
| Bacdanol (IFF) | A | A | A | A |
| Kephalis (Givaudan) | A | A | A | A |
| Levosandol (Takasago) | A | A | A | A |
| Mayol (Firmenich) | A | A | A | A |
| Nerolidol | A | A | A | A |
| Raspberry ketone | A | A | A | A |
| Apharmate (IFF) | A | A | A | A |
| p-Methoxyphenethyl alcohol | A | A | A | A |
| Thesaron (Takasago) | A | A | A | A |
| Dihydrodamascone | A | A | A | A |
| Tansy oil | A | A | A | A |
| Basil oil | A | A | A | A |

As is evident from the results of Table 6, all preparations of the present invention are excellent in terms of stability and have high masking effect.

### Example 4

A hair color second preparation was prepared by the formulation shown in Table 7. In this connection, illustrative example of the deodorant composition in Table 7 are shown in Table 8.

**Table 7**

| Compound | Weight % |
|---|---|
| Phenacetin | 0.1 |
| Diphenyl ether | 0.2 |
| Ethanol (95%) | 2.0 |
| Citric acid | proper amount |
| Hydrogen peroxide (35%) | 17.0 |
| Propylene glycol | 5.0 |
| Deodorant composition | 0.2 |
| Purified water | balance |

(Evaluation) Masking effect of the hair color second preparation and stability of its aroma were evaluated by the following evaluation method.

### (Evaluation method)

### (5) Masking effect

The hair dye second preparation was stored at 37°C for 12 weeks, and then its masking effect was evaluated by a sensory test based on the following criteria.
A; very good, B; good, C; slightly bad, D; bad

### (6) Stability of aroma and masking effect

A hair dye treatment was carried out by a conventional method using the hair color second preparation.

(6-a) Stability effect: Changing degree of the aromatic tone at the bottle mouth when the container was opened before the treatment was evaluated by a sensory test based on the following criteria.
A; no change, B; slightly changed, C; fairly changed, D; greatly changed

(6-b) Masking effect: A smell from hair during the treatment (just after application to hair) and after the treatment was evaluated by a sensory test based on the following criteria.
A; very good, B; slightly good, C; usual or slightly bad, D; bad

Results of the evaluation by the evaluation methods are shown in Table 8. In this connection, the "masking effect (5)" in the table is an evaluation result by the evaluation method (5), the "stability of aroma" is an evaluation result by the evaluation method (6-a), and other "masking effect" is an evaluation result by the evaluation method (6-b).

**Table 8**

| Compound | Masking Effect (5) | Stability of aroma | Masking effect | |
|---|---|---|---|---|
| | | | during treatment | after treatment |
| Koavone (IFF) | A | A | A | A |
| Linalool oxide | A | A | A | A |
| Rosephenone | A | A | A | A |
| Sandalmysore core (Kao) | A | A | A | A |
| Sandalore (Givaudan) | A | A | A | A |
| Bacdanol (IFF) | A | A | A | A |
| Kephalis (Givaudan) | A | A | A | A |
| Mayol (Firmenich) | A | A | A | A |
| Nerolidol | A | A | A | A |
| Raspberry ketone | A | A | A | A |
| Apharmate (IFF) | A | A | A | A |
| Thesaron (Takasago) | A | A | A | A |
| Dihydrodamascone | A | A | A | A |
| Tansy oil | A | A | A | A |
| Basil oil | A | A | A | A |

As shown in Table 8, the deodorant composition of the present invention was highly stable even in the hair color second preparation (acidic, pH = 2), masked the ammoniacal smell and also showed high stability even after subsequent hair color treatment by a conventional method, showing no changes in aroma.

As is evident from these results, the deodorant composition of the present invention has a superior effect in masking the unpleasant smell originated from ammonia contained in the hair color first preparation and the double preparation type hair bleach first preparation, is excellent in terms of the stability of aroma of the original substance even under a highly alkaline condition (pH 8 to 11), and also has a superior effect in masking the unpleasant smell originated from ammonia even after the treatment of hair. In addition, it was confirmed that it is excellent in terms of the stability of aroma of the original substance even under a strongly acidic condition (pH 2) and also has a superior effect in masking the unpleasant smell even after the treatment of hair.

Also, as shown in Table 8, it is evident that the deodorant composition of the present invention is highly stable even in the hair color second preparation (pH = 2, acidic), has the effect in masking the ammoniacal smell, is also stable even in the acidic base material which is used thereafter or simultaneously, and hardly shows changes in aroma.

While the invention has been described in detail and with reference to specific embodiments thereof, it will be apparent to one skilled in the art that various changes and modifications can be made therein without departing from the scope thereof.

This application is based on Japanese patent application No. 2002-080167 filed March 22, 2002, the entire contents thereof being hereby incorporated by reference.

## Claims

1. A deodorant composition for hair color or hair bleach, which comprises at least one compound or essential oil selected from acetyl diisoamylene, linalol oxide, rosephenone, 2-methyl-4-(2,2,3-trimethyl-3-cyclopenten-1-yl)-2-buten-1-ol, 3-methyl-5-(2,2,3-trimethyl-3-cyclopenten-1-yl)-pentan-2-ol, 2-ethyl-4-(2,2,3-trimethyl-3-cyclopenten-1-yl)-2-buten-1-ol, 4-(1-ethoxyvinyl)-3,3,5,5-tetramethylcyclohexanone, cis-p-menthan-7-ol, nerolidol, raspberry ketone, α,3,3-trimethylcyclohexanemethyl formate, p-methoxyphenethyl alcohol, 2,2,6-trimethylcyclohexanecarboxylic acid ethyl ester, 2,2,6-trimethyl-1-crotonylcyclohexane, tansy oil and basil oil.

2. A deodorant composition for hair color or hair bleach, which comprises
at least one compound or essential oil selected from acetyl diisoamylene, linalol oxide, rosephenone, 2-methyl-4-(2,2,3-trimethyl-3-cyclopenten-1-yl)-2-buten-1-ol, 3-methyl-5-(2,2,3-trimethyl-3-cyclopenten-1-yl)-pentan-2-ol, 2-ethyl-4-(2,2,3-trimethyl-3-cyclopenten-1-yl)-2-buten-1-ol, 4-(1-ethoxyvinyl)-3,3,5,5-tetramethylcyclohexanone, cis-p-menthan-7-ol, nerolidol, raspberry ketone, α,3,3-trimethylcyclohexanemethyl formate, p-methoxyphenethyl alcohol, tansy oil and basil oil as component A, and
at least one compound selected from 2,2,6-trimethylcyclohexanecarboxylic acid ethyl ester and 2,2,6-trimethyl-1-crotonylcyclohexane as component B.

3. A hair color composition or hair bleach composition comprising a deodorant composition according to claim 1 and a permeation-promoting agent.

4. A hair color composition or hair bleach composition comprising a deodorant composition according to claim 2 and a permeation-promoting agent.

5. A hair color composition or hair bleach composition comprising a deodorant composition according to claim 1 and an oxidizing agent, wherein said hair color composition or hair bleach composition being adjusted to pH 2 to 4.

6. A hair color composition or hair bleach composition comprising a deodorant composition according to claim 2 and an oxidizing agent, wherein said hair color composition or hair bleach composition being adjusted to pH 2 to 4.

7. A hair color composition or hair bleach composition comprising:
a first agent comprising a permeation-promoting agent and
a second agent comprising an oxidizing agent and being adjusted to pH 2 to 4,
wherein either one or both of the first agent and the second agent comprises a deodorant composition according to claim 1.

8. A hair color composition or hair bleach composition comprising:
a first agent comprising a permeation-promoting agent and
a second agent comprising an oxidizing agent and being adjusted to pH 2 to 4,
wherein either one or both of the first agent and the second agent comprises a deodorant composition according to claim 2.

9. A method for deodorizing the odor during and after hair coloring or hair bleaching, using a deodorant composition according to claim 1.

10. A method for deodorizing the odor during and after hair coloring or hair bleaching, using a deodorant composition according to claim 2.
